# EUROPEAN PATENT SPECIFICATION

(11) **EP 2 712 636 B1**
(45) Date of publication and mention of the grant of the patent: **16.11.2016**
(21) Application number: 12186093.6
(22) Date of filing: 26.09.2012
(51) Int. Cl.: A61M 1/10, A61M 1/12

(54) **A pediatric heart assist pump**
Herzunterstützungspumpe für Kinder
Pompe cardiac assistante pour enfants

(43) Date of publication of application: 02.04.2014
(73) Proprietor: Fundacja Rozwoju Kardiochirurgii Im. Prof. Zbigniewa Religi, 41-800 Zabrze (PL)
(72) Inventor: Kustosz, Roman, 41-800 Zabrze (PL); Kapis, Artur, 41-800 Zabrze (PL)
(74) Representative: Malcherek, Piotr

(56) References cited:
- WO-A1-00/35515
- WO-A1-90/07648
- GB-A- 2 245 937
- JP-A- H 025 966
- US-A- 15 192
- US-A- 4 573 883
- US-A- 4 782 817
- US-A- 4 838 889
- US-A- 4 888 011
- US-A- 5 643 172
- US-A1- 2012 130 484

## Description

The present invention relates to a paediatric heart pump used in treating heart failures in children.

There are heart pumps destined for use in children. An example of such pump may be the device disclosed in the patent description US2006/0199993A.

The most similar device known from the prior art is a paediatric heart pump available on the market comprising a blood chamber and a pneumatic chamber separated by a membrane. An inflow connector for blood and an outflow connector for blood are connected to the blood chamber. Furthermore, the chamber comprises an inflow valve and an outflow valve for regulating blood flow through the blood chamber, which flow is caused by an alternate movement of the membrane under a wave of pneumatic pressure delivered to the pneumatic chamber. In the known paediatric pump artificial heart valves are applied, in particular trilobate, polyurethane valves. Due to the necessity to miniaturise the heart pump if it is applied in children, the valves also have smaller dimensions, which leads to problems connected with insufficient rinsing of the valve leaflets, both from the chamber side and from the connectors' side, as well as the possibility of blood pools and thrombosis.

The closest prior art is US 4838889. This document discloses a heart pump comprising a blood chamber and a pumping chamber wherein an inflow connector for blood and an outflow connector of blood are connected to the blood chamber. The pump also comprises an inflow valve and an outflow valve for regulating blood flow through the blood chamber, wherein the inflow valve has a form of a single leaflet, which leaflet is situated around an opening of the blood chamber connecting said chamber with the inflow connector from the side of the blood chamber. The leaflet is an extension of the internal surface of the wall of the arterial blood chamber situated between the inflow connector and the outflow connector.

The aim of the invention is to devise a paediatric heart pump which would eliminate the problems known from the state of the art.

Another aim of the invention is to ensure the most favourable blood flow inside the pump at each stage of the pump's work.

Another aim of the invention is to stabilize and achieve repeatability of the inflow valve movement of the pump.

The aims of the invention listed above have been achieved by the pump constructed according to the enclosed claims, including the independent claim no. 1. The essence of the invention in particular consists in that the inflow valve in a paediatric pump is a single leaflet with varying thickness, preferably made of polyurethane, which leaflet is situated around the opening of the blood chamber connecting the chamber with the inflow connector from the side of the blood chamber, wherein the leaflet is the extension of the internal surface of the chamber's wall situated between the inflow connector and the outflow connector. The thickness of the leaflet diminishes towards the free, loose end thereof and the thickness of the leaflet at the free end thereof is 45 - 60 % of the thickness of the leaflet at the base thereof. Further the leaflet has a skeleton in the form of an internal supporting structure, whereas the external surfaces of the leaflet are smooth.

It is preferable that the leaflet is attached to the pump around 65 - 75% of the circumference thereof.

It is also recommendable that the blood chamber is asymmetrical in relation to the axis thereof and in the area of blood inflow has smaller radiuses of curvature of the internal surface than the radiuses of curvature of the outflow area from the blood chamber having a form of a funnel taking up 60 - 70% of the volume of the blood chamber.

In construction of the pump according to the invention one leaflet has been applied as a heart valve, which leaflet is not only a blood flow regulating valve but also a guide for blood flow both during blood inflow to the pipe and during blood circulation in the blood chamber of the pump. Preferably the leaflet is made of polyurethane, which means of the same biocompatible material of which blood chambers of pumps are most often made. The leaflet is elastic and is of varying thickness, and, moreover, has an internal skeleton in the form of a supporting structure, which allows to stabilize the work thereof and obtain repeatability of the leaflet movement during work of the pump. The leaflet is attached tangentially to the internal surface of the chamber, in particular the surface of the leaflet from the side of the blood chamber is tangent to the internal surface of the blood chamber around a part of the edge thereof in the area of connection of the inflow connector with the blood chamber. The other free, unbound part of the leaflet circumference allowed it to open and let blood flow into the blood chamber. Owing to this construction of the inflow valve, during ejection of the blood from the pump, the surface of the leaflet will naturally close the inflow connector while maintaining the tangency of the leaflet surface to the internal surface of the blood chamber. Therefore, the leaflet constitutes a wall of the blood chamber during the outflow of blood from said chamber. During blood inflow to the blood chamber the single leaflet valve opens thus allowing blood inflow to the blood chamber and during the whole phase of filling the leaflet is simultaneously naturally rinsed both from the side of the inflow connector - by the bloodstream flowing in - and from the side of the leaflet - by the swirling bloodstream created during circulation of blood entering inside the blood chamber. Owing to the wave of bloodstream flowing tangentially to the circumference of the blood chamber the most favourable conditions are provided for rinsing the corner area of the membrane assembly fitting. The use of the polyurethane single leaflet valve entirely eliminates the "dead" volume of blood lingering between the typically used mechanical valve and the inflow connector stub connected to the blood chamber, it also completely eliminates the risk of thrombosis material creation in line of the valve fitting. Another benefit of this solution is the fact that the material for constructing the valve is biocompatible polyurethane, the same as used in the whole prosthesis, owing to which the valve will ensure a perfect continuity of the surface and the structure of the valve will be integrated with the blood inflow connector leading to the blood chamber. Moreover, the internal skeleton structure protects the leaflet against bending during the closing phase and forces the desired shape during the opening phase. The most advantageous work parameters of the inflow valve in the form of a leaflet are obtained if an asymmetrical blood chamber is applied. The shape of the chamber was devised in order to ensure the most favourable blood flow inside the chamber during all work phases thereof. The volume of blood flowing into the chamber maintains tangency of the inflow bloodstream in relation to the circumference of the blood chamber. The internal shape of the chamber allows for effective rinsing thereof by the stream, both inside and near the walls. Furthermore, the indicated proportions of diminishing thickness of the leaflet as well as proportions of the circumference around which the leaflet is attached to the walls of the chamber determine most favourable parameters of effective work of the leaflet as an inflow valve.

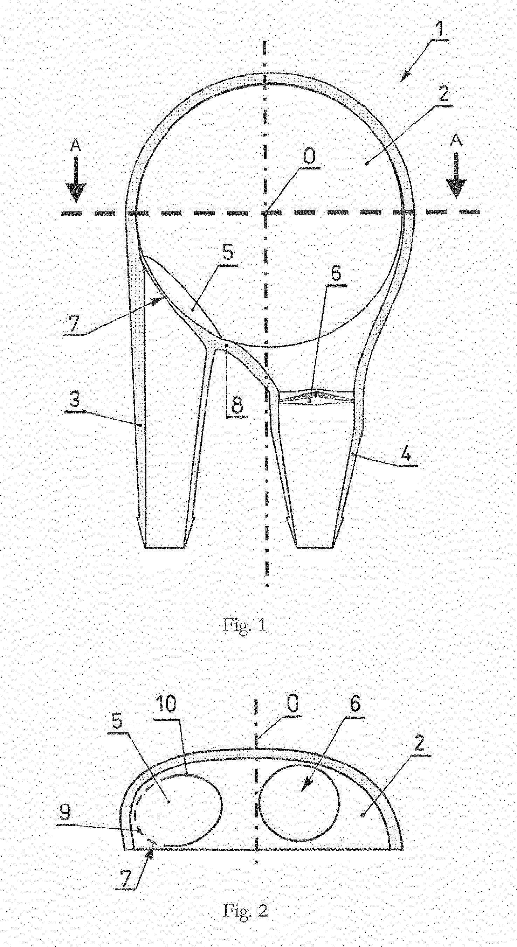

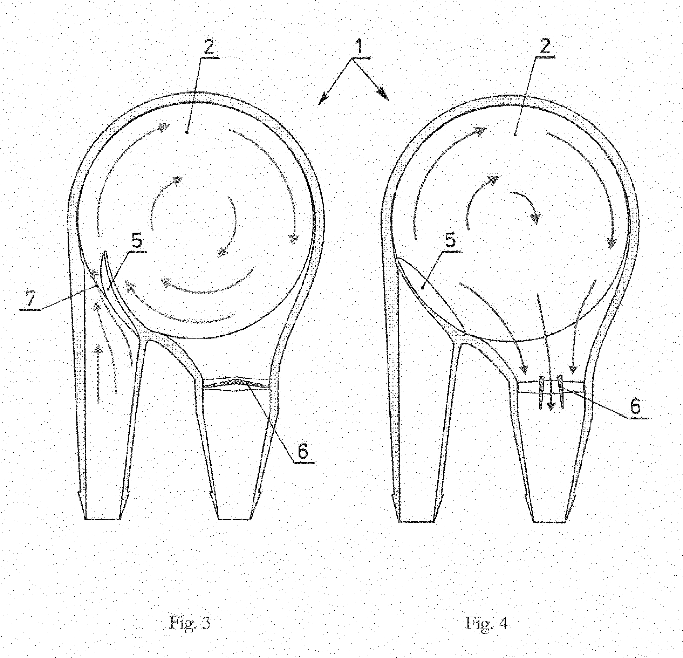

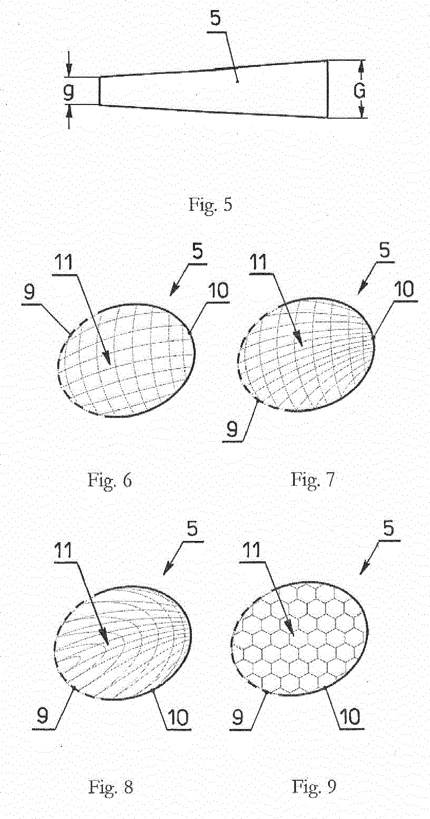

The invention is presented in more detail in the following embodiment and in the enclosed drawings, where fig. 1 is a longitudinal section of the pump, fig. 2 - an A-A section of fig.1, fig. 3 and fig. 4 - cross-sections of the pump during the phase of blood flowing into the pump and during the phase of blood flowing out of the pump with a schematically shown direction of bloodstream, fig.5 is a schematic longitudinal section of the leaflet as an inflow valve, fig. 6 to fig. 9 are different embodiments of the leaflet as an inflow valve in a schematic section showing the internal skeleton of said leaflet.

A paediatric heart pump 1 is destined to be used as a pump implantable into a patient's body and comprises a blood chamber 2 and a pneumatic chamber not shown in the drawing. Said chambers are separated by a membrane whose alternate movement caused by the delivery of a pneumatic wave into the pneumatic chamber results in forcing the movement of the membrane assembly, thus causing blood to flow through the blood chamber 2. An inflow connector 3 for blood and an outflow connector 4 for blood are connected to the blood chamber 2. Moreover, the pump 1 comprises an inflow valve 5 and an outflow valve 6 for regulating the blood flow through the blood chamber 2. The inflow valve 5 has a form of a leaflet with varying thickness, made of polyurethane, wherein in other embodiments the leaflet can be made of a different plastic used in medicine which is biocompatible with blood. The leaflet being the inflow valve 5 is situated from the side of the blood chamber 2 around an opening 7 in the blood chamber 2, which connects the chamber 2 with the inflow connector 3. The leaflet is an extension of the internal surface of the wall 8 of the chamber 2 situated between the inflow connector 3 and the outflow connector 4. The thickness of the leaflet diminishes towards the free, loose end thereof (fig. 5). For example, the thickness G of the leaflet at its base is 0.6 mm, and the thickness g at the free end thereof is 0.3 mm. In general the thickness g at the free end thereof is in the range between 45-60% of the thickness G of the leaflet at the base thereof. The most advantageous work characteristics of the leaflet is obtained when said leaflet is attached leaflet has an internal skeleton 11 in the form of a supporting structure. In fig. 6 to fig. 9 different types of the skeleton 11 are shown, in particular in the form of a mesh with differently shaped elements. The external surfaces of the leaflet are smooth, without unevenness. The blood chamber 2 is asymmetrical in relation to the axis 0 thereof and in the area of the inflow of blood has smaller radiuses of curvature of the internal surface thereof than the radiuses of curvature of the area of the outflow of blood from the blood chamber 2 shaped as a funnel taking up 60-70% of the volume of the blood chamber 2.

An artificial double-disc valve is used as an outflow valve 6, however, it is possible to use a different mechanical valve, for example single-disc valves.

## Claims

1. A paediatric heart pump comprising a blood chamber ( 2 ) and a pneumatic chamber, wherein to the blood chamber ( 2 ) are connected an inflow connector ( 3 ) for blood and an outflow connector ( 4 ) for blood, and also comprising an inflow valve ( 5 ) and an outflow valve ( 6 ) for regulating blood flow through the blood chamber ( 2 ), **characterised in that** the inflow valve ( 5 ) has a form of a leaflet of varying thickness preferably made of polyurethane, which leaflet is situated around an opening ( 7 ) of the blood chamber ( 2 ) connecting the chamber ( 2 ) with the inflow connector ( 3 ) from the side of the blood chamber ( 2 ), wherein the leaflet is an extension of the internal surface of a wall ( 8 ) of the blood chamber ( 2 ) situated between the inflow connector ( 3 ) and the outflow connector ( 4 ), and the thickness of the leaflet diminishes towards the free end thereof, and the thickness ( g ) of the leaflet at the free end thereof is 45-60% of the thickness ( G ) of the leaflet at the base thereof, and the leaflet has a skeleton ( 11 ) in the form of an internal supporting structure, and the external surfaces of the leaflet are smooth.

2. The paediatric heart pump according to claim 1, **characterised in that** the leaflet is attached to the pump around 65-75% of the circumference thereof.

3. The paediatric heart pump according to claim 1 or 2, **characterised in that** the blood chamber ( 2 ) is asymmetrical in relation to the axis (0) thereof and in the area of the inflow of blood has smaller radiuses of curvature of the internal surface thereof than the radiuses of curvature of the area of the outflow of blood from the blood chamber ( 2 ) in the shape of a funnel taking up 60 - 70% of the volume of the blood chamber (2).

## Patentansprüche

1. Pädiatrische Herz-Assist-Pumpe, welche eine Blutkammer ( 2 ) und eine pneumatische Kammer enthält, wobei an die Blutkammer ( 2 ) ein Blutzufuhrverbindungselement ( 3 ) und ein Blutausflussverbindungselement ( 4 ) angeschlossen sind; sowie eine Zufuhrklappe ( 5 ) und eine Ausflussklappe ( 6 ) für Blutflussregelung durch die Blutkammer ( 2 ) enthält, **dadurch gekennzeichnet, dass** die Zufuhrklappe ( 5 ) in Form eines Blättchens variabler Dicke, vorteilhaft aus Polyurethan, ausgerührt ist, und dass das Blättchen rund um eine Öffnung ( 7 ) der Blutkammer ( 2 ) gelegt ist, die diese Blutkammer ( 2 ) mit dem Blutzufuhrverbindungselement ( 3 ) von Seite der Blutkammer ( 2 ) verbindet, wobei das Blättchen eine Verlängerung der inneren Oberfläche einer Wand ( 8 ) der zwischen dem Blutzufuhrverbindungselement ( 3 ) und dem Blutausflussverbindungselement platzierten Blutkammer ( 2 ) bildet, und die Dicke des Blättchens nimmt in Richtung seiner freien Ende ab, wobei die Dicke ( g ) des Blättchens an seinem freien Ende 45-60% der Dicke ( G ) des Blättchens an seiner Grundfläche beträgt, und das Blättchen ein Gerüst in Form einer inneren Tragkonstruktion besitzt, und die äußeren Oberflächen des Blättchens glatt sind.

2. Pädiatrische Herz-Assist-Pumpe nach Anspruch 1 **dadurch gekennzeichnet, dass** das Blättchen rund um 65-75% seines Umfangs an Pumpe befestigt ist.

3. Pädiatrische Herz-Assist-Pumpe nach Anspruch 1 oder 2 **dadurch gekennzeichnet, dass** die Blutkammer ( 2 ) asymmetrisch in Bezug auf Achse ( O ) ist, und ihre innere Oberfläche im Blutzustrom-Bereich kleinere Krümmungsradien aufweist, als Krümmungsradien in ihrem Blutausfluss-Bereich, wobei der Blutausfluss-Bereich in Trichterform gestaltet ist und 60-70% des Volumens der Blutkammer ( 2 ) umfasst.

## Revendications

1. Pédiatrique pompe de soutien cardiaque contenant une chambre sanglante (2) et une chambre pneumatique pendant qu'à la chambre sanglante (2) sont connectés un connecteur d'entrée (3) pour le sang et un connecteur de sortie (4) pour le sang et qui contient la valve d'entrée (5) et la valve de sortie (6) pour la réglementation de la circulation du sang par la chambre sanglante (2) **caractérisée en ce que** la valve d'entrée (5) possède une forme d'un pétale à l'épaisseur variable fait favorablement du polyuréthane, le dit pétale est localisé autour de l'ouverture (7) de la chambre sanglante (2) qui relie cette chambre (2) au connecteur d'entrée (3) du coté de la chambre sanglante (2), pandant que le pétale est un prolongement de la surface intérieure de paroi (8) de la chambre sanglante (2) situé entre le connecteur d'entrée (3) et le connecteur de sortie (4) tandis que l'épaisseur du pétale se réduit vers son extrémité libre pendant que l'épaisseur (g) du pétale à son extrémité libre est de 45-60% de l'épaisseur (G) du pétale à sa base et le pétale possède un squelette (11) en forme d'une structure de soutien interne et les surfaces externes sont lisses.

2. Pédiatrique pompe de soutien cardiaque selon la revendication 1 **caractérisée en ce que** le pétale est monté à la pompe autour de 65-75% de son pourtour.

3. Pédiatrique pompe de soutien cardiaque selon la revendication 1 ou 2 **caractérisée en ce que** la chambre sanglante (2) est asymétrique par rappor à son axe (0) et dans la zone de l'entreé du sang elle a plus petits rayons de courbure de la surface intérieure que les rayons de courbure de la zone de la sortie du sang de la chambre sanglante (2) formée en trémie occupant 60-70% du volume de la chambre sanglante (2).
